(19) Europäisches Patentamt
European Patent Office
Office européen des brevets

(11) Numéro de publication : **0 388 254 B1**

(12) # FASCICULE DE BREVET EUROPEEN

(45) Date de publication du fascicule du brevet : **02.12.92 Bulletin 92/49**

(51) Int. Cl.$^5$ : **C08F 220/06,** A61L 15/00

(21) Numéro de dépôt : **90400607.9**

(22) Date de dépôt : **06.03.90**

(54) **Polymère hydrophile à base d'acide acrylique et d'acrylate de métal alcalin, son procédé de préparation et son application comme agent absorbant, en particulier comme agent absorbant pour articles d'hygiène.**

(30) Priorité : **16.03.89 FR 8903488**

(43) Date de publication de la demande : **19.09.90 Bulletin 90/38**

(45) Mention de la délivrance du brevet : **02.12.92 Bulletin 92/49**

(84) Etats contractants désignés : **AT BE CH DE DK ES FR GB GR IT LI LU NL SE**

(56) Documents cités :
**EP-A- 0 295 784**
**DE-A- 3 239 476**
**FR-A- 2 606 414**

(73) Titulaire : **SOCIETE FRANCAISE HOECHST**
**TOUR ROUSSEL HOECHST 1 Terrasse Bellini**
**F-92800 Puteaux (FR)**

(72) Inventeur : **Mallo, Paul**
**290 avenue Napoléon Bonaparte**
**F-92500 Rueil Malmaison (FR)**
Inventeur : **Moreau, Marie-Thérèse**
**7 résidence Leblond**
**F-95350 Saint Brice Sous Foret (FR)**
Inventeur : **Cabestany, Jean**
**127 boulevard Maxime Gorki**
**F-93240 Stains (FR)**

(74) Mandataire : **Rinuy, Santarelli**
**14, avenue de la Grande Armée**
**F-75017 Paris (FR)**

**EP 0 388 254 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un polymère hydrophile à haute capacité d'absorption sous charge de solutions aqueuses salines, à base d'acide acrylique et d'acrylate de métal alcalin, son procédé de préparation et son application comme agent absorbant, notamment comme agent absorbant pour couches pour bébés.

On connait des substances polymères susceptibles d'absorber plusieurs fois leur propre poids d'eau en se transformant en gel. Ces polymères hydrophiles sont soit des polymères naturels ou semi-synthétiques tels que des dérivés de la cellulose, de l'amidon, de l'alginate, de polysaccharides, soit des polymères synthétiques à base notamment d'acide maléique ou d'acide (méth)acrylique.

On connaît ainsi une grande variété de polymères hydrophiles mais pour des usages agricoles, horticoles ou sanitaires, on recherche toujours des produits présentant une capacité d'absorption élevée non seulement pour l'eau, mais également pour de l'eau chargée en électrolytes, avec une vitesse d'absorption rapide et un bon pouvoir de rétention à l'état de gel.

Par ailleurs, pour l'usage hygiénique, notamment dans la fabrication d'objets destinés à être en contact, à l'état humide, avec l'épiderme humain, on recherche des produits atoxiques, peu onéreux, ne présentant pas de synérèse, ne contenant ni dérivés azotés ni monomères résiduels et possédant simultanément une capacité d'absorption élevée sans charge et sous charge de solutions aqueuses salines. En effet, par exemple, un des problèmes importants à résoudre dans la fabrication des couches pour bébés, est la rétention de l'urine par la couche, pendant plusieurs heures, à des températures pouvant atteindre 40°C et malgré la pression due au poids du bébé. Une solution partielle à ces problèmes a été apportée par l'enseignement du brevet européen N° 0.083.022 qui préconise une post-réticulation de la résine absorbant l'eau avec un agent réticulant ayant au moins deux groupes fonctionnels qui peuvent réagir avec les groupements carboxylate ou les groupements présents dans cette résine à groupement carboxylate, tels que des groupements hydroxyle, amino ou sulfo en présence de 0,01 à 1,3 partie en poids d'eau par partie en poids de la résine. Cette solution, quoique intéressante, est onéreuse à mettre en oeuvre : elle exige, en effet, de préparer dans un premier stade, la résine absorbant l'eau puis, dans un deuxième stade, de soumettre cette résine à une post-réticulation dans des conditions particulières.

En conclusion, on peut dire aujourd'hui qu'aucun des polymères hydrophiles connus absorbant l'eau salée ne répond aux besoins exprimés par le marché.

Or la Demanderesse a découvert de façon surprenante un tel produit. La présente invention concerne donc un polymère hydrophile, en microperles, insoluble dans l'eau, à base d'acide acrylique et d'acrylate de métal alcalin, caractérisé en ce qu'il est constitué par un copolymère acide acrylique-acrylate de potassium, contenant en proportions molaires de 55 à 85 % d'acrylate de potassium, réticulé avec de 100 ppm à 3000 ppm par rapport au poids total des monomères d'un ou plusieurs réticulants choisis dans le groupe constitué par les produits de formule générale I :

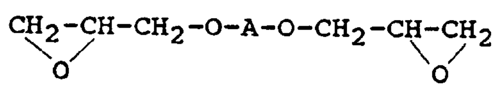

(I)

dans laquelle A représente un radical bivalent dérivant d'un alcane en $C_3$-$C_6$, ramifié ou non, par enlèvement d'un atome d'hydrogène à chacun des deux atomes de carbone terminaux tels que les radicaux triméthylène, tétraméthylène, éthyléthylène, propylène, diméthyl-2,2-propanediyl-1,3 et qu'il présente une capacité d'absorption d'une solution physiologique salée de l'ordre de 30 à 70 g par gramme et de l'ordre de 10 à 35 g par gramme sous une pression de 15 g par $cm^2$.

Par "microperles", on entend des perles sensiblement sphériques de diamètre compris entre 0,05 et 1 mm.

Par "insolubles dans l'eau", on entend que les polymères renferment, à la température ambiante, moins de 5 % de produits solubles dans l'eau.

Ces copolymères sont de préférence réticulés avec 200 à 3000 ppm et notamment 200 à 1000 ppm par rapport au poids des monomères de bis(oxiranylméthoxy)-1,4-butane, désigné couramment diglycidyléther du butanediol-1,4.

Parmi les copolymères définis ci-dessus, on peut citer plus particulièrement les copolymères acide acrylique-acrylate de potassium contenant en proportions molaires de 70 à 75 % d'acrylate de potassium.

Par "solution physiologique salée", on désigne une solution isotonique contenant 9 g de chlorure de sodium par litre d'eau distillée.

Selon l'invention, les copolymères définis ci-dessus sont susceptibles d'être préparés par un procédé de polymérisation en suspension eau dans huile réalisé en atmosphère inerte, caractérisé en ce que l'on introduit

lentement, sous agitation, dans une phase huile désoxygénée, maintenue à l'ébullition et contenant un colloïde protecteur, une phase aqueuse contenant le ou les agents de réticulation de formule générale (I), un ou plusieurs amorceurs de polymérisation hydrosolubles générateurs de radicaux libres, les monomères choisis et de l'eau en quantité telle que la concentration en monomères soit de 55 ± 10 % en poids, puis lorsque la réaction de polymérisation est terminée, l'on élimine par distillation azéotropique 75 à 90 % de l'eau introduite et l'on isole ensuite le polymère formé, soit par filtration suivie d'un séchage à 110 ± 10°C pendant au moins 30 minutes, soit par séchage azéotropique direct à 110 ± 10°C pendant au moins 30 minutes de manière à obtenir des microperles du polymère hydrophile cherché contenant pondéralement 7 ± 3 % d'eau.

Selon une variante du procédé décrit ci-dessus, on peut placer le ou les agents de réticulation de formule générale (I) utilisés en tout ou partie dans la phase huile.

Par "huile désoxygénée", l'on entend une huile presque parfaitement ou de préférence parfaitement désoxygénée.

La phase huile est constituée principalement par un ou plusieurs hydrocarbures, non miscibles à l'eau, inertes vis-à-vis des amorceurs de polymérisation et susceptibles de donner un azéotrope avec l'eau, tels que le cyclohexane ou les fractions d'essence de points d'ébullition compris entre 50 et 180°C.

Le rapport pondéral phase huile sur phase aqueuse est avantageusement compris entre 0,9 et 1,1.

La réaction de polymérisation est réalisée au reflux du milieu réactionnel, habituellement à la pression ambiante. Elle peut également être réalisée à une pression inférieure ou supérieure à la pression ambiante.

Le colloïde protecteur de préférence utilisé à la dose de 0,5 à 2 % en poids par rapport au poids de monomères est choisi parmi ceux couramment utilisés dans ce type de polymérisation en suspension (cf. Kirk-Othmer, Encyclopedia of Chemical Technology, 3ème édition, volume 1, page 400). Avantageusement, on choisira un éther de cellulose et, préférentiellement, un éthyléther de cellulose présentant un taux d'éthoxyle de 48 à 49,5 % (cf. Encyclopedia of Polymer Science and Engineering, 2ème édition, volume 3, page 254). Le colloïde protecteur est préalablement dissous ou dispersé dans la phase huile.

La réaction de polymérisation est amorcée avec un ou plusieurs générateurs de radicaux libres, hydrosolubles, présentant une demi-vie à 70°C supérieure à deux heures. Avantageusement, la réaction de polymérisation est amorcée avec un mélange d'au moins deux agents amorceurs hydrosolubles différents tels que définis précédemment dont au moins un est choisi parmi les peroxydes minéraux, et au moins un est choisi parmi les dérivés azoïques. Ils sont avantageusement utilisés à la concentration de 200 à 3000 ppm par rapport au poids des monomères et avantageusement de 500 à 1000 ppm. De tels agents d'amorçage sont notamment certains peroxydes minéraux comme le peroxodisulfate de sodium ou certains azoïques comme l'acide dicyano-4,4′-azo-4,4′ dipentanoïque. L'agent ou les agents d'amorçage utilisés sont dissous dans de l'eau puis cette solution est soigneusement désoxygénée.

L'acrylate de potassium est obtenu avantageusement en solution aqueuse, par salification directe d'une solution aqueuse d'acide acrylique avec de la potasse. Cette salification est avantageusement effectuée à une température comprise entre 20 et 35°C. Les monomères utilisés sont dissous dans de l'eau, de préférence à une concentration d'environ 55 ± 10 % en poids.

La solution aqueuse du ou des amorceurs et le milieu aqueux contenant en solution les monomères, et éventuellement le ou les agents de réticulation choisis, sont avantageusement introduits dans la phase huile après mélange extemporané au fur et à mesure de leur introduction, de manière qu'ils ne restent en contact que quelques secondes.

La durée d'introduction peut varier selon les unités opératoires, mais généralement elle est comprise entre une et deux heures. En fin d'introduction, il est avantageux de maintenir le milieu réactionnel à l'ébullition sous agitation pour parfaire la polymérisation.

Au cours de la réaction de polymérisation, les copolymères formés se réticulent mutuellement spontanément pour fournir des copolymères réticulés insolubles dans l'eau, à fort pouvoir hydrophile et à très faibles taux de monomères résiduels, lesquels sont toujours inférieurs à 0,01 % en poids par rapport au poids du polymère.

La réticulation spontanée est d'autant plus favorisée que le degré de neutralisation de l'acide acrylique est plus faible et que la température de polymérisation est plus élevée. Les copolymères de la présente invention sont donc réticulés, d'une part par voie thermique, et d'autre part par voie chimique par le ou les agents de réticulation de formule générale (I).

Afin de développer toutes ses propriétés, le polymère hydrophile selon la présente invention doit être séché au moins pendant 30 minutes à une température de 110 ± 10°C, de manière à obtenir un séchage homogène à l'intérieur même des microperles.

Il est très étonnant qu'un polymère hydrophile présente vis-à-vis de l'eau salée une capacité d'absorption élevée, aussi bien tel quel que sous l'influence d'une pression de 15 g/cm$^2$ car, pour l'homme du métier, ces deux propriétés sont antinomiques. Il est connu, en effet, que pour obtenir un polymère hydrophile présentant

vis-à-vis de l'eau salée une capacité d'absorption élevée sous contrainte, il faut que ce polymère soit fortement réticulé, afin de ne pas s'écraser et, réciproquement, une capacité d'absorption élevée à l'état tel quel est obtenue avec un polymère très faiblement réticulé. Or les polymères hydrophiles de la présente invention possèdent simultanément ces deux propriétés, ce qui indique que les microperles du polymère sont faiblement réticulées en surface. On peut donc dire que les microperles des polymères selon la présente invention présentant une structure "core-shell" (noyau-coquille).

La capacité d'absorption d'eau du polymère est déterminée à 20°C, en agitant durant 30 minutes 0,4 g de polymère dans 500 g d'eau, puis en pesant le gel polymère obtenu égoutté. Le poids trouvé est ramené à 1 g de polymère. Les copolymères de la présente invention présentent dans ce test une capacité d'absorption de l'ordre de 250 à 750 g par gramme.

La capacité d'absorption de solution physiologique salée du polymère est déterminée à 20°C, en agitant durant 30 minutes 2 g de polymère dans 500 g de solution physiologique salée, puis en pesant le gel polymère obtenu égoutté. Le poids trouvé est ramené comme précédemment à 1 g de polymère. Les copolymères de la présente invention présentent dans ce test une capacité d'absorption de l'ordre de 30 à 70 g par gramme.

La vitesse d'absorption de solution physiologique salée du polymère est déterminée à 20°C, dans un bécher de 100 ml, de diamètre 55 mm et de hauteur 70 mm, en agitant à la vitesse de 600 tours par minute avec un agitateur magnétique équipé d'un barreau aimanté de 25 mm de longueur, 2 g de polymère dans 50 g de solution physiologique salée et en mesurant le temps nécessaire à la disparition du vortex. Dans ce test, les copolymères de la présente invention demandent 50 à 80 secondes pour la disparition du vortex.

Le taux d'extractibles est déterminé selon la méthode suivante :

- on place 1 g de polymère à tester dans 200 g de solution physiologique salée ;
- on agite cette suspension 16 heures à 20°C, puis on la filtre ;
- on dose sur 100 $cm^3$ du filtrat les fonctions carboxyliques et carboxylates présentes ;
- on exprime le résultat de ce dosage en gramme de polymère dissous pour 100 g de polymère sec.

Dans ce test, les copolymères de la présente invention présentent un taux d'extractibles de 1 à 5 %. En fin de réaction de polymérisation, on élimine par distillation azéotropique les solvants réactionnels jusqu'à obtention d'une suspension présentant un taux de siccité de 80 ± 10 % puis la suspension est filtrée et le précipité recueilli et séché à 90-95 % de siccité.

La capacité d'absorption par capillarité sous une charge de 15 g par $cm^2$ est déterminée à 20°C selon le protocole suivant : dans un entonnoir cylindrique à plaque filtrante de diamètre 90 mm et de porosité 1, on étale successivement et uniformément, 40 g de sable de Fontainebleau de granulométrie 0,100 à 0,300 mm, 2 g de copolymère à tester et enfin 40 g de sable de Fontainebleau. On place ensuite sur a couche supérieure du sable par l'intermédiaire d'un disque de verre de diamètre 90 mm, une charge totale de 954 g, puis l'entonnoir est plongé dans un bac contenant une solution physiologique salée, à niveau constant, de manière que le niveau de l'eau affleure rigoureusement la face supérieure du fritté et on mesure la quantité de solution physiologique salée absorbée par capillarité par le copolymère en 90 minutes.

On exprime le résultat en g de solution physiologique salée par gramme de polymère sec.

L'extrait sec est déterminé par séchage d'un échantillon à poids constant à 140°C et il est exprimé en pourcentage pondéral de produit sec contenu dans le produit brut.

Les copolymères de la présente invention présentent donc d'intéressantes propriétés absorbantes qui justifient leur application comme agent absorbant. L'invention a donc également pour objet les polymères tels que définis précédemment, comme composants absorbants, de préférence dans un article d'hygiène, notamment pour la fabrication de couches pour bébés, d'articles pour incontinents ou de serviettes périodiques.

Les exemples suivants illustrent l'invention sans toutefois la limiter :

## Exemple 1

On disperse en atmosphère inerte dans 618,5 g (7,35 moles) de cyclohexane : 3,5 g d'éthyléther de cellulose, contenant de 48 à 49,5 % de groupements éthoxylés et présentant à 25°C, une viscosité de 200 mPa.s déterminée en solution à 5 % dans un mélange toluène-éthanol 80-20 en poids.

Dans cette dispersion, soigneusement désoxygénée, agitée et maintenue à l'ébullition, on introduit en 90 minutes, en atmosphère inerte, une solution obtenue par mélange extemporané au cours de l'introduction :

- d'une part, d'une solution désoxygénée de :
  - . 0,106 g de peroxodisulfate de sodium,
  - . 0,212 g d'acide dicyano-4,4'-azo-4,4' dipentanoïque dissous dans 10 g de potasse 0,2 N ;
- d'autre part, d'une solution préparée extemporanément :
  - . en dissolvant à une température inférieure à 30°C : 230 g (3,192 moles) d'acide acrylique dans 417,4 g d'une solution aqueuse de potasse contenant 128,9 g (2,297 moles) d'hydroxyde de potassium,

. puis en ajoutant dans cette solution 319 mg de bis (oxiranylméthoxy)-1,4 butane.

La suspension obtenue est ensuite maintenue une heure à l'ébullition sous agitation, puis elle est soumise à une distillation azéotropique avec recyclage du cyclohexane jusqu'à élimination d'environ 85 % de l'eau présente, et enfin elle est refroidie à la température ambiante et filtrée. Le précipité recueilli est ensuite séché à 110°C pendant 60 minutes dans une étuve ventilée. On obtient ainsi un polymère hydrophile sous forme de microperles blanches, insoluble dans l'eau, présentant les caractéristiques suivantes :

- extrait sec : 92 % ;
- capacité d'absorption d'eau : 330 g par gramme ;
- capacité d'absorption d'une solution physiologique salée :
  . tel quel : 44 g/gramme,
  . sous une pression de 15 g/cm$^2$ : 23 g/gramme;
  . taux d'extractibles : 2 % en poids,
- taux de monomères résiduels : inférieur à 0,005 % en poids ;
- vitesse d'absorption d'une solution physiologique salée : 70 s ;
- granulation : 0,1 - 0,8 mm.

Exemple 2

On opère comme dans l'exemple 1 mais en utilisant 208 mg au lieu de 319 mg de bis(oxiranylméthoxy)-1,4 butane.

On obtient ainsi un polymère hydrophile, en microperles, présentant les propriétés suivantes :

- extrait sec : 92 % ;
- capacité d'absorption d'eau : 380 g par gramme ;
- capacité d'absorption d'une solution physiologique salée :
  . tel quel : 47 g/gramme,
  . sous une pression de 15 g/cm$^2$ : 26 g/gramme;
- taux de monomères résiduels : inférieur à 0,005 % en poids.

Exemple 3

On opère comme dans l'exemple 1 mais en utilisant 159 mg au lieu de 319 mg de bis(oxiranylméthoxy)-1,4 butane.

On obtient ainsi un polymère hydrophile, en microperles, présentant les propriétés suivantes :

- extrait sec : 92 % ;
- capacité d'absorption d'eau : 485 g par gramme ;
- capacité d'absorption d'une solution physiologique salée :
  . tel quel : 54,5 g/gramme,
  . sous une pression de 15 g/cm$^2$ : 27,5 g/gramme;
- taux d'extractibles : 1,6 % ;
- taux de monomères résiduels : inférieur à 0,005 % en poids.

Exemple 4

On opère comme dans l'exemple 1 mais on utilise 300 mg au lieu de 319 mg de bis(oxiranylméthoxy)-1,4 butane, que l'on place dans la phase huile.

On obtient ainsi un polymère hydrophile, en microperles, présentant les propriétés suivantes :

- extrait sec : 92 % ;
- capacité d'absorption d'eau : 340 g par gramme ;
- capacité d'absorption d'une solution physiologique salée :
  . tel quel : 46,5 g/gramme,
  . sous une pression de 15 g/cm$^2$ : 24 g/gramme;
- taux de monomères résiduels : inférieur à 0,005 % en poids.

Exemple 5

On opère comme dans l'exemple 1 mais on utilise 256 mg au lieu de 319 mg de bis(oxiranylméthoxy)-1,4 butane, dont 128 mg sont placés dans la phase huile et 128 mg sont placés dans la phase aqueuse contenant les monomères.

On obtient ainsi un polymère hydrophile, en microperles, présentant les propriétés suivantes :
- extrait sec : 92 % ;
- capacité d'absorption d'eau : 320 g par gramme ;
- capacité d'absorption d'une solution physiologique salée :
  . tel quel : 46 g/gramme,
  . sous une pression de 15 g/$cm^2$ : 27 g/gramme ;
- taux de monomères résiduels : inférieur à 0,005 % en poids.

Il va de soi que la présente invention n'a été décrite qu'à titre purement explicatif et nullement limitatif et que toute modification, notamment au niveau des équivalences techniques, pourra y être apportée sans sortir de son cadre.

## Revendications

### Revendications pour les Etats contractants suivants : AT, BE, CH, DE, DK, FR, GB, GR, IT, LI, LU, NL, SE

**1.** Polymère hydrophile, en microperles, insoluble dans l'eau, à base d'acide acrylique et d'acrylate de métal alcalin, caractérisé en ce qu'il est constitué par un copolymère acide acrylique-acrylate de potassium, contenant en proportions molaires de 55 à 85 % d'acrylate de potassium, réticulé avec de 100 ppm à 3000 ppm par rapport au poids total des monomères d'un ou plusieurs réticulants choisis dans le groupe constitué par les produits de formule générale I :

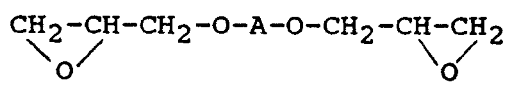

(I)

dans laquelle A représente un radical bivalent dérivant d'un alcane en $C_3$-$C_6$, ramifié ou non, par enlèvement d'un atome d'hydrogène à chacun des deux atomes de carbone terminaux tels que les radicaux triméthylène, tétraméthylène, éthyléthylène, propylène, diméthyl-2,2-propanediyl-1,3 et qu'il présente une capacité d'absorption d'une solution physiologique salée de l'ordre de 30 à 70 g par gramme et de l'ordre de 10 à 35 g par gramme sous une pression de 15 g par $cm^2$.

**2.** Polymère hydrophile selon la revendication 1, caractérisé en ce qu'il est réticulé avec 200 ppm à 3000 ppm de bis(oxiranylméthoxy)-1,4 butane.

**3.** Polymère hydrophile selon la revendication 2, caractérisé en ce qu'il est réticulé avec 200 ppm à 1000 ppm de bis(oxiranylméthoxy)-1,4 butane.

**4.** Polymère hydrophile selon l'une quelconque des revendications 1 à 3, caractérisé en ce qu'il contient en proportions molaires de 70 à 75 % d'acrylate de potassium.

**5.** Procédé de préparation d'un polymère hydrophile selon la revendication 1 par polymérisation en suspension eau dans huile réalisé en atmosphère inerte, caractérisé en ce que l'on introduit lentement, sous agitation, dans une phase huile désoxygénée, maintenue à l'ébullition et contenant un colloïde protecteur, une phase aqueuse contenant le ou les agents de réticulation de formule générale (I), un ou plusieurs amorceurs de polymérisation hydrosolubles générateurs de radicaux libres, les monomères choisis et de l'eau en quantité telle que la concentration en monomères soit de 55 ± 10 % en poids, puis lorsque la réaction de polymérisation est terminée, l'on élimine par distillation azéotropique 75 à 90 % de l'eau introduite et l'on isole ensuite le polymère formé, soit par filtration suivie d'un séchage à 110 ± 10°C pendant au moins 30 minutes, soit par séchage azéotropique direct à 110 ± 10°C pendant au moins 30 minutes de manière à obtenir des microperles du polymère hydrophile cherché contenant pondéralement 7 ± 3 % d'eau.

**6.** Procédé selon la revendication 5, caractérisé en ce que l'on place le ou les agents de réticulation de formule générale (I) en tout ou partie dans la phase huile.

**7.** Procédé selon l'une quelconque des revendications 5 et 6, caractérisé en ce que l'on amorce la polymérisation par un mélange d'au moins deux agents amorceurs hydrosolubles différents, présentant une durée de demi-vie à 70°C supérieure à 2 heures, dont au moins un est choisi parmi les peroxydes minéraux et au moins un est choisi parmi les agents amorceurs hydrosolubles azoïques.

**8.** Utilisation d'un polymère hydrophile selon l'une quelconque des revendications 1 à 4, comme composant absorbant, de préférence dans un article d'hygiène.

**Revendications pour l'Etat contractant suivant : ES**

**1.** Polymère hydrophile, en microperles, insoluble dans l'eau, à base d'acide acrylique et d'acrylate de métal alcalin, caractérisé en ce qu'il est constitué par un copolymère acide acrylique-acrylate de potassium, contenant en proportions molaires de 55 à 85 % d'acrylate de potassium, réticulé avec de 100 ppm à 3000 ppm par rapport au poids total des monomères d'un ou plusieurs réticulants choisis dans le groupe constitué par les produits de formule générale I :

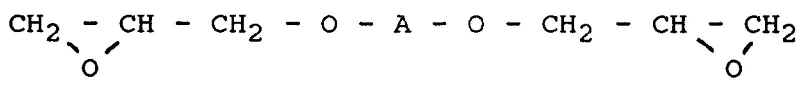

(I)

dans laquelle A représente un radical bivalent dérivant d'un alcane en $C_3$-$C_6$, ramifié ou non, par enlèvement d'un atome d'hydrogène à chacun des deux atomes de carbone terminaux tels que les radicaux triméthylène, tétraméthylène, éthyléthylène, propylène, diméthyl-2,2-propanediyl-1,3 et qu'il présente une capacité d'absorption d'une solution physiologique salée de l'ordre de 30 à 70 g par gramme et de l'ordre de 10 à 35 g par gramme sous une pression de 15 g par $cm^2$.

**2.** Polymère hydrophile selon la revendication 1, caractérisé en ce qu'il est réticulé avec 200 ppm à 3000 ppm de bis (oxyranylméthoxy)-1,4 butane.

**3.** Polymère hydrophile selon la revendication 2, caractérisé en ce qu'il est réticulé avec 200 ppm à 1000 ppm de bis (oxyranylméthoxy)-1,4 butane.

**4.** Polymère hydrophile selon l'une quelconque des revendications 1 à 3, caractérisé en ce qu'il contient en proportions molaires de 70 à 75 % d'acrylate de potassium.

**5.** Procédé de préparation d'un polymère hydrophile, en microperles, insoluble dans l'eau, à base d'acide acrylique et d'acrylate de métal alcalin, constitué par un copolymère acide acrylique-acrylate de potassium, contenant en proportions molaires de 55 à 85 % d'acrylate de potassium, réticulé avec de 100 ppm à 3000 ppm par rapport au poids total des monomères d'un ou plusieurs réticulants choisis dans le groupe constitué par les produits de formule générale I :

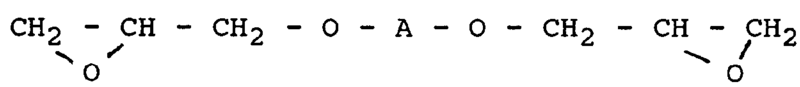

(I)

dans laquelle A représente un radical bivalent dérivant d'un alcane en $C_3$-$C_6$, ramifié ou non, par enlèvement d'un atome d'hydrogène à chacun des deux atomes de carbone terminaux tels que les radicaux triméthylène, tétraméthylène, éthyléthylène, propylène, diméthyl-2,2-propanediyl-1,3 et qu'il présente une capacité d'absorption d'une solution physiologique salée de l'ordre de 30 à 70 g par gramme et de l'ordre de 10 à 35 g par gramme sous une pression de 15 g par $cm^2$, par polymérisation en suspension eau dans huile réalisé en atmosphère inerte, dans lequel on introduit lentement, sous agitation, dans une phase huile désoxygénée, maintenue à l'ébullition et contenant un colloïde protecteur, une phase aqueuse contenant le ou les agents de réticulation de formule générale (I), un ou plusieurs amorceurs de polymérisation hydrosolubles générateurs de radicaux libres, les monomères choisis et de l'eau en quantité telle que la concentration en monomère soit de 55 ± 10 % en poids, puis lorsque la réaction de polymérisation est terminée, l'on élimine par distillation azéotropique 75 à 90 % de l'eau introduite et l'on isole ensuite le polymère formé, soit par filtration suivie d'un séchage à 110 ± 10 °C pendant au moins 30 minutes, soit par séchage azéotropique direct à 110 ± 10°C pendant au moins 30 minutes de manière à obtenir des microperles du polymère hydrophile cherché contenant pondéralement 7 ± 3 % d'eau.

6. Procédé selon la revendication 5, caractérisé en ce que l'on place le ou les agents de réticulation de formule générale (I) en tout ou partie dans la phase huile.

7. Procédé selon l'une quelconque des revendications 5 et 6, caractérisé en ce que l'on amorce la polymérisation par un mélange d'au moins deux agents amorceurs hydrosolubles différents, présentant une durée de demi-vie à 70°C supérieure à 2 heures, dont au moins un est choisi parmi les péroxydes minéraux et au moins un est choisi parmi les agents amorceurs hydrosolubles azoïques.

8. Utilisation d'un polymère hydrophile selon l'une quelconque des revendications 1 à 4 ou obtenu selon l'une quelconque des revendications 5 à 7, comme composant absorbant, de préférence dans un article d'hygiène.

## Patentansprüche

### Patentansprüche für folgende Vertragsstaaten : AT, BE, DK, FR, GB, GR, IT, LU, NL, DE, SE, CH

1. Hydrophiles Polymer in Form von Mikroperlen, das in Wasser unlöslich ist, auf Basis von Acrylsäure und eines Alkalimetallacrylats, dadurch gekennzeichnet, daß es aus einem Acrylsäure-Kaliumacrylatcopolymer besteht, 55 bis 85 Mol-% Kaliumacrylat enthält und mit 100 bis 3000 ppm, bezogen auf das Gesamtgewicht der Monomere, eines odere mehrerer Vernetzungsmittel ausgewählt aus der Gruppe bestehend aus den Produkten der allgemeinen Formel (I)

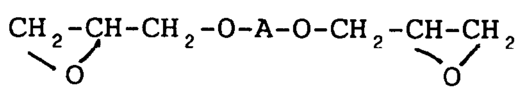

(I),

worin A einen zweiwertigen Rest darstellt, der von einem gegebenenfalls verzweigten $C_3$-$C_6$-Alkan durch Entfernen eines Wasserstoffatoms an jedem der beiden endständigen Kohlenstoffatome abgeleitet ist, wie die Trimethylen-, Tetramethylen-, Äthyläthylen-, Propylen- und Dimethyl-1,3-propandiyl-Reste, vernetzt ist und daß es ein Absorptionsvermögen einer physiologischen Kochsalzlösung von 30 bis 70 g pro g und von 10 bis 35 g pro g unter einem Druck von 15 g pro $cm^2$ aufweist.

2. Hydrophiles Polymer nach Anspruch 1, dadurch gekennzeichnet, daß es mit 200 bis 3000 ppm 1,4-Bis-(oxiranylmethoxy)-butan vernetzt ist.

3. Hydrophiles Polymer nach Anspruch 2, dadurch gekennzeichnet, daß es mit 200 bis 1000 ppm 1,4-Bis-(oxiranylmethoxy)-butan vernetzt ist.

4. Hydrophiles Polymer nach einem der Ansprüche 1 bis 3, dadurch gekennzeichnet, daß es 70 bis 75 Mol-% Kaliumacrylat enthält.

5. Verfahren zur Herstellung eines hydrophilen Polymers nach Anspruch 1 durch Polymerisation in einer Wasser-in-Öl-Suspension, die in inerter Atmosphäre durchgeführt wird, dadurch gekennzeichnet, daß in eine siedende desoxygenierte Ölphase, die ein Schutzkolloid enthält, langsam unter Rühren eine wässerige Phase, die das oder die Vernetzungsmittel der allgemeinen Formel (I), ein oder mehrere wasserlösliche, freie Radikale erzeugende Polymerisationsinitiatoren, die gewählten Monomere und Wasser enthält, in einer solchen Menge eingebracht wird, daß die Konzentration der Monomere 55 ± 10 Gew.% beträgt, worauf, wenn die Polymerisationsreaktion beendet ist, 75 bis 90 % des eingebrachten Wassers durch azeotrope Destillation entfernt werden und danach das gebildete Polymer durch Filtration und nachfolgendes Trocknen bei 110 ± 10°C während mindestens 30 Minuten oder durch direktes azeotropes Trocknen bei 110 ± 10°C während mindestens 30 Minuten isoliert wird, um Mikroperlen des gewünschten hydrophilen Polymers zu erhalten, die 7 ± 3 Gew.% Wasser enthalten.

6. Verfahren nach Anspruch 5, dadurch gekennzeichnet, daß das oder die Vernetzungsmittel der allgemeinen Formel (I) auf einmal oder portionsweise in die Ölphase gegeben werden.

7. Verfahren nach einem der Ansprüche 5 und 6, dadurch gekennzeichnet, daß die Polymerisation durch eine Mischung von mindestens zwei verschiedenen wasserlöslichen Initiatoren, die eine Halbwertzeit bei 70°C von mehr als 2 Stunden aufweisen, wovon mindestens einer ausgewählt ist aus den mineralischen Peroxiden und mindestens einer ausgewählt ist aus den wasserlöslichen Azoinitiatoren, eingeleitet wird.

8. Verwendung eines hydrophilen Polymers nach einem der Ansprüche 1 bis 4 als Absorptionsmittel, vorzugsweise in einem Sanitärartikel.

**Patentansprüche für folgenden Vertragsstaat : ES**

1. Hydrophiles Polymer in Form von Mikroperlen, das in Wasser unlöslich ist, auf Basis von Acrylsäure und eines Alkalimetallacrylats, dadurch gekennzeichnet, daß es aus einem Acrylsäure-Kaliumacrylatcopolymer besteht, 55 bis 85 Mol-% Kaliumacrylat enthält und mit 100 bis 3000 ppm, bezogen auf das Gesamtgewicht der Monomere, eines odere mehrerer Vernetzungsmittel ausgewählt aus der Gruppe bestehend aus den Produkten der allgemeinen Formel (I)

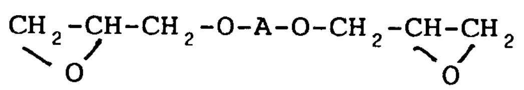 (I),

worin A einen zweiwertigen Rest darstellt, der von einem gegebenenfalls verzweigten $C_3$-$C_6$-Alkan durch Entfernen eines Wasserstoffatoms an jedem der beiden endständigen Kohlenstoffatome abgeleitet ist, wie die Trimethylen-, Tetramethylen-, Äthyläthylen-, Propylen- und 2,2-Dimethyl-1,3-propandiyl-Reste, vernetzt ist, und daß es ein Absorptionsvermögen einer physiologischen Kochsalzlösung von 30 bis 70 g pro g und von 10 bis 35 g pro g unter einem Druck von 15 g pro $cm^2$ aufweist.

2. Hydrophiles Polymer nach Anspruch 1, dadurch gekennzeichnet, daß es mit 200 bis 3000 ppm 1,4-Bis-(oxiranylmethoxy)-butan vernetzt ist.

3. Hydrophiles Polymer nach Anspruch 2, dadurch gekennzeichnet, daß es mit 200 bis 1000 ppm 1,4-Bis-(oxiranylmethoxy)-butan vernetzt ist.

4. Hydrophiles Polymer nach einem der Ansprüche 1 bis 3, dadurch gekennzeichnet, daß es 70 bis 75 Mol-% Kaliumacrylat enthält.

5. Verfahren zur Herstellung eines hydrophilen Polymers in Form von Mikroperlen, das in Wasser unlöslich ist, auf Basis von Acrylsäure und eines Alkalimetallacrylats, das aus einem Acrylsäure-Kaliumacrylatcopolymer besteht, 55 bis 85 Mol-% Kaliumacrylat enthält und mit 100 bis 3000 ppm, bezogen auf das Gesamtgewicht der Monomere, eines odere mehrerer Vernetzungsmittel ausgewählt aus der Gruppe bestehend aus den Produkten der allgemeinen Formel (I)

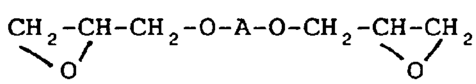 (I),

worin A einen zweiwertigen Rest darstellt, der von einem gegebenenfalls verzweigten $C_3$-$C_6$-Alkan durch Entfernen eines Wasserstoffatoms an jedem der beiden endständigen Kohlenstoffatome abgeleitet ist, wie die Trimethylen-, Tetramethylen-, Äthyläthylen-, Propylen- und 2,2-Dimethyl-1,3-propandiyl-Reste, vernetzt ist und ein Absorptionsvermögen einer physiologischen Kochsalzlösung von 30 bis 70 g pro g und von 10 bis 35 g pro g unter einem Druck von 15 g pro $cm^2$ aufweist, durch Polymerisation in einer Wasser-in-Öl-Suspension, die in inerter Atmosphäre durchgeführt wird, dadurch gekennzeichnet, in welchem in eine siedende desoxygenierte Ölphase, die ein Schutzkolloid enthält, langsam unter Rühren eine wässerige Phase, die das oder die Vernetzungsmittel der allgemeinen Formel (I), ein oder mehrere wasserlösliche, freie Radikale erzeugende Polymerisationsinitiatoren, die gewählten Monomere und Wasser enthält, in einer solchen Menge eingebracht wird, daß die Konzentration der Monomere 55 ± 10 Gew.% beträgt, worauf, wenn die Polymerisationsreaktion beendet ist, 75 bis 90 % des eingebrachten Wassers durch azeotrope Destillation entfernt werden und danach das gebildete Polymer durch Filtration und nachfolgendes Trocknen bei 110 ± 10°C während mindestens 30 Minuten oder durch direktes azeotropes

Trocknen bei 110 ± 10°C während mindestens 30 Minuten isoliert wird, um Mikroperlen des gewünschten hydrophilen Polymers zu erhalten, die 7 ± 3 Gew.% Wasser enthalten.

6. Verfahren nach Anspruch 5, dadurch gekennzeichnet, daß das oder die Vernetzungsmittel der allgemeinen Formel (I) auf einmal oder portionsweise in die Ölphase gegeben werden.

7. Verfahren nach einem der Ansprüche 5 und 6, dadurch gekennzeichnet, daß die Polymerisation durch eine Mischung von mindestens zwei verschiedenen wasserlöslichen Initiatoren eingeleitet wird, die eine Halbwertzeit bei 70°C von mehr als 2 Stunden aufweisen, wovon mindestens einer ausgewählt ist aus den mineralischen Peroxiden und mindestens einer ausgewählt ist aus den wasserlöslichen Azoinitiatoren.

8. Verwendung eines hydrophilen Polymers nach einem der Ansprüche 1 bis 4 oder erhalten nach einem der Ansprüche 5 bis 7 als Absorptionsmittel, vorzugsweise in einem Sanitärartikel.

## Claims

### Claims for the following Contracting States : AT, BE, DK, FR, GB, GR, IT, LU, NL, DE, SE, CH

1. A water-insoluble hydrophilic polymer in microbeads on the basis of acrylic acid and alkali metal acrylate, characterized in that it is formed by an acrylic acid/potassium acrylate copolymer containing in molar proportions from 55 to 85% potassium acrylate crosslinked with 100 ppm to 3000 ppm in relation to the total weight of the monomers of one or more crosslinkage agents selected from the group formed by the products having the general Formula I:

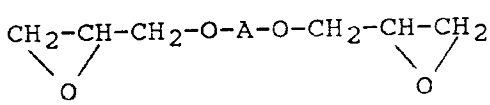

(I)

where A denotes a bivalent radical deriving from a branched or unbranched $C_3$-$C_6$ alkane by the removal of one atom of hydrogen from each of the two terminal carbon atoms such as the trimethylene, tetramethylene, ethyl ethylene, propylene, 2,2-dimethyl-,3-propanediyl, said hydrophilic polymer having the capacity of absorption of a saline physiological solution of the order of 30 to 70 g per gram and of the order of 10 to 35 g per gram at a pressure of 15 g per $cm^2$.

2. A hydrophilic polymer according to claim 1, characterized in that it is crosslinked with 200 ppm to 3000 ppm of 1,4-bis (oxiranylmethoxy) butane.

3. A hydrophilic polymer according to claim 2, characterized in that it is crosslinked with 200 ppm to 1000 ppm of 1,4-bis-(oxiranylmethoxy) butane.

4. A hydrophilic polymer according to any of claims 1 to 3, characterized in that it contains in molar proportions from 70 to 75% potassium acrylate.

5. A process for the preparation of a hydrophilic polymer according to claim 1 by polymerization in water-in-oil suspension performed in an inert atmosphere, characterized in that an aqueous phase containing the or each crosslinkage agent having the general formula (I), one or more water-soluble polymerization initiators which generate free radicals, the selected monomers and water in a quantity such that the concentration of monomers is 55 ± 10% by weight are introduced slowly with agitation into a deoxygenated oil phase maintained at boiling and containing a protective colloid, whereafter the polymerization reaction is terminated, 75 to 90% of the water introduced is eliminated by azeotropic distillation and then the polymer formed is isolated, either by filtration followed by a drying at 110 ± 10°C for at least 30 minutes, or by direct azeotropic drying at 110 ± 10°C for at least 30 minutes, so as to obtain microbeads of the required hydrophilic polymer containing 7 ± 3% by weight of water.

6. A process according to claim 5, characterized in that the or each crosslinkage agent having the general Formula (I) is placed wholly or partially in the oil phase.

7. A process according to either of claims 5 and 6, characterized in that polymerization is initiated by a mixture of at least two different water-soluble initiator agents having a half-life greater than 2 hours at 70°C, at least one of the agents being selected from the mineral peroxides and at least one being selected from the azo water-soluble initiator agents.

8. Use of a hydrophilic polymer according to any of claims 1 to 4 as an absorptive component, preferably in an article for hygiene.

**Claims for the following Contracting State : ES**

1. A water-insoluble hydrophilic polymer in microbeads on the basis of acrylic acid and alkali metal acrylate, characterized in that it is formed by an acrylic acid/potassium acrylate copolymer containing in molar proportions from 55 to 85% potassium acrylate crosslinked with 100 ppm to 3000 ppm in relation to the total weight of the monomers of one or more crosslinkage agents selected from the group formed by the products having the general Formula I:

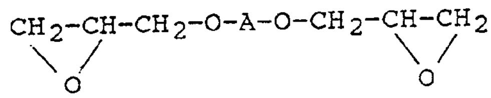

(I)

where A denotes a bivalent radical deriving from a branched or unbranched $C_3$-$C_6$ alkane by the removal of one atom of hydrogen from each of the two terminal carbon atoms such as the trimethylene, tetramethylene, ethyl ethylene, propylene, 2,2-dimethyl-1,3-propanediyl, said hydrophilic polymer having the capacity of absorption of a saline physiological solution of the order of 30 to 70 g per gram and of the order of 10 to 35 g per gram at a pressure of 15 g per $cm^2$.

2. A hydrophilic polymer according to claim 1, characterized in that it is crosslinked with 200 ppm to 3000 ppm of 1,4-bis (oxiranylmethoxy) butane.

3. A hydrophilic polymer according to claim 2, characterized in that it is crosslinked with 200 ppm to 1000 ppm of 1,4-bis-(oxiranylmethoxy) butane.

4. A hydrophilic polymer according to any of claims 1 to 3, characterized in that it contains in molar proportions from 70 to 75% potassium acrylate.

5. A water-insoluble hydrophilic polymer in microbeads on the basis of acrylic acid and alkali metal acrylate, characterized in that it is formed by an acrylic acid/potassium acrylate copolymer containing in molar proportions from 55 to 85% potassium acrylate crosslinked with 100 ppm to 3000 ppm in relation to the total weight of the monomers of one or more crosslinkage agents selected from the group formed by the products having the general Formula I:

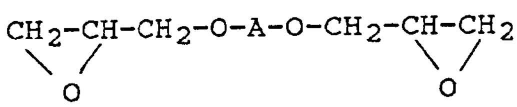

(I)

where A denotes a bivalent radical deriving from a branched or unbranched $C_3$-$C_6$ alkane by the removal of one atom of hydrogen from each of the two terminal carbon atoms such as the trimethylene, tetramethylene, ethyl ethylene, propylene, 2,2-dimethyl-1,3-propanediyl, said hydrophilic polymer having the capacity of absorption of a saline physiological solution of the order of 30 to 70 g per gram and of the order of 10 to 35 g per gram at a pressure of 15 g per $cm^2$, by polymerization in a water-in-oil suspension performed in an inert atmosphere, wherein an aqueous phase containing the or each crosslinkage agent having the general Formula (I), one or more water-soluble polymerization initiators which generate free radicals, the selected monomers and water in a quantity such that the concentration of monomers is 55 ± 10% by weight are introduced slowly with agitation into a deoxygenated oil phase maintained at boiling and containing a protective colloid, whereafter the polymerization reaction is terminated, 75 to 90% of the water introduced is eliminated by azeotropic distillation and then the polymer formed is isolated, either by filtration followed

by a drying at 110 ± 10°C for at least 30 minutes, or by direct azeotropic drying at 110 ± 10°C for at least 30 minutes, so as to obtain microbeads of the required hydrophilic polymer containing 7 ± 3% by weight of water.

**6.** A process according to claim 5, characterized in that the or each crosslinkage agent having the general Formula (I) is placed wholly or partially in the oil phase.

**7.** A process according to either of claims 5 and 6, characterized in that polymerization is initiated by a mixture of at least two different water-soluble initiator agents having a half-life greater than 2 hours at 70°C, at least one of the agents being selected from the mineral peroxides and at least one being selected from the azo water-soluble initiator agents.

**8.** Use of a hydrophilic polymer according to any of claims 1 to 4 or obtained according to any of claims 5 to 7 as an absorptive component, preferably in an article for hygiene.